# NEW EUROPEAN PATENT SPECIFICATION

(11) **EP 3 005 948 B2**
(45) Date of publication and mention of the opposition decision: **01.11.2023**
(45) Mention of the grant of the patent: 17.10.2018
(21) Application number: 15158482.8
(22) Date of filing: 10.03.2015
(51) Int. Cl.: A61B 10/00, A63H 27/10, A61B 5/155, A61B 5/15, B65B 3/00

(54) **APPARATUS, SYSTEM AND METHOD FOR FILLING CONTAINERS WITH FLUIDS**
VORRICHTUNG, SYSTEM UND VERFAHREN ZUM BEFÜLLEN VON BEHÄLTERN MIT FLÜSSIGKEITEN
APPAREIL, SYSTÈME ET PROCÉDÉ DE REMPLISSAGE DE RÉCIPIENTS AVEC DES FLUIDES

(30) Priority: 07.02.2014 US 201461937083 P; 20.02.2014 US 201461942193 P; 22.09.2014 US 201414492487
(43) Date of publication of application: 13.04.2016
(60) Divisional application: 18175805.3
(73) Proprietor: Tinnus Enterprises, LLC, Plano, TX 75074 (US)
(72) Inventor: Malone, Joshua, Plano, TX Texas 75074 (US)
(74) Representative: Dentons UK and Middle East LLP

(56) References cited:
- CN-Y- 201 161 115
- CN-Y- 201 161 115
- FR-A1- 2 911 512
- FR-A1- 2 911 512
- GB-A- 2 344 057
- US-A- 179 236
- US-A- 600 967
- US-A- 1 703 463
- US-A- 3 580 303
- US-A- 5 014 757
- US-A- 5 127 867
- US-A- 5 127 867
- US-A1- 2005 004 430
- US-A1- 2008 121 309
- US-A1- 2013 118 640
- US-A1- 2013 118 640
- US-A1- 2013 226 219
- US-A1- 2013 226 219
- US-B1- 6 488 557
- US-B1- 6 488 557
- Anonymous: "Bunch O Balloons: 100 Water Balloons in Less Than 1 Minute by Tinnus Enterprises - Kickstarter", , 26 July 2014 (2014-07-26), XP055407607, Retrieved from the Internet: URL:https://web.archive.org/web/2014072604 0504/https://www.kickstarter.com/projects/ bunchoballoons/bunch-o-balloons-100-water- balloons-in-less-than-1 [retrieved on 2017-09-18]
- eyeSight Mobile Technologies Ltd.: "ControAir App", You Tube, 21 January 2015 (2015-01-21), page 1, XP054977751, Retrieved from the Internet: URL:https://www.youtube.com/watch?v=fJtjVc r3qtc [retrieved on 2017-09-22]
- Oxford Dictionary inflatable
- Oxford Dictionary sealing
- reminder PCT deadline
- public prior use New York Toy Fair 1/4
- public prior use New York Toy Fair 2/4
- public prior use New York Toy Fair 3/4
- public prior use New York Toy Fair 4/4
- public prior use Kickstarter
- video Kickstarter on YouTubehttps://www.youtube.co m/watch?v=JxrGNE78y-o
- upload date YouTube video
- screenshot video Kickstarter on YouTube
- screenshot video Kickstarter on YouTube.
- screenshot video Kickstarter on YouTube..
- Kickstarter pages (Copy filed, see also:https://www.kickstarter com/proiects/burchoballoons/bunch-o-Hoons- 100-water-balloons-in-less.-than-l)
- Internet article ABC news (Copy filed, see also: http,'7abcnews qo.com/lifestyle/father-creates-Qenius-wat er-balloon-invention/storv?id=24698486)
- Movie published on the Kickstarter page and on ABC News(on DVD)
- Movie by NBC's 'TODAY', posted on YouTube (see:https :/www.youtube.com/watch?v=OQL5SAkqi98 (on DVD)
- Screenshot from YouTube page showing D25
- Movie posted by LuckyPennyShop.com on YouTube (see:https://www.voutube.com/watch?v=S1DaX YT6Q2A)(on DVD)
- Screenshot from YouTube page showing D27
- Internet article CNN Money (Copy filed, see:http://monev.cnn.com/2014/07/29/smallb usiness/bunch-o-balloons-kickstarter/index .html)
- Letter of 18 August 2016 by UKIPO examiner in GB 2525306
- Assignment of US 61/937083
- Assignment of US 61/942193
- Front page WO 2008/024519 (PCT/US2007/018941)
- Letter supporting restoration request priority year, with attachments, filed 4 January 2016
- Gleysteen M.D., J J. (2016). 'Review article. A history of mtragastnc balloons.' Surgery for Obesity and Related Diseases, 12, 430-435.
- 'Intragastric Balloon?. Bray, G.A. & Bouchard, C. (2014) Handbook of Obesity. Clinical Applications (Volume 2. Fourth Edition. Pages 422-423). Boca Raton, USA: CRC Press.
- Zubrowski, Bernie, "Balloons. Building and experimenting with inflatable toys" HarperTrophy, 1990 ISBN- 978-0688083243
- Debrun, M.D., G. et al., "Endovascular Occlusion of Vertebral Fistulae by Detachable Balloons with Conservation of the Vertebral Blood Flow" Radiology, Vol. 130, No. Issue l, 1979
- Salter?s Chemistry Club, ?HYDROGEN BALLOONS 06.11.2007, [cited 24.07.2020] Available from: [http://resources.schoolscience.co.uk/Salt ers/pdfs/volone/SCC1 Prac21 .pdf]
- Wayback machine (Internet Archive), "Hydrogen Balloons", [cited25.07.2020] Available from:[https://web.archive.org/web/20071106 215307/http://resources.schoolscience.co.u k/Salters/pdfs/volone/SCC1_ Prac21 pdf]
- Questacon, "Model of a lung" 24.08.2011, [cited 27.07.2020] Available from: [https://youtu.be/CBv2BqqAydE]
- Sarah, "Home Wine-making Getting Started" 21.01.2013, [cited22.08.2020] Available from: [http://arwz.com/ssblog/2013/01/21/home¬wi ne-making-getting-started/]
- Central Board of Secondary Education, "Biology. Unit-1. Life Processes" Delhi, India: CBSE-lnternational, 02.06.2012
- Ruhland, Pela, Coopers Home Brew Kit 18.03.2008, [cited12.07.2020] Available from: [https://www.coroflot.com/PetaRuhland'Coop ers-Home-Brew-Kit-Uni]
- Cake and Commerce. "Making sausage with a food processor and a pastry bag" 26.04.2009, [cited 12.07.2020] Available from: [https://cakeandcommerce.typepad.com/cake_ and_commerce/2009/04/sausage-making-tips.h tml]
- Parker, Louise, "The OPAL DIY Weather Kit" 02.03.2011. [cited24.07.2020] Available from:[https/www.slnnr.org.uk/documents/pro jects/opal/Weather%20kit.pdf]

## Description

### TECHNICAL FIELD

The present disclosure relates generally to fluid inflatable systems and more particularly, to an apparatus, system and method for filling containers with fluids.

### BACKGROUND

Inflatable containers such as balloons can be filled with a variety of fluids, such as air, helium, water, medicines, etc. In some cases, it may be desirable for a lot of inflatable containers to be filled with fluids. For example, festive balloons used as props in conventions, large parties, etc. may number in the hundreds and may require substantial human effort to fill them all in a timely manner. In another example, water balloons used as kids' toys may need to be filled in large numbers to aid in various games. Various methods may be employed to fill such inflatable containers. For example, an individual may sequentially blow up and tie each balloon by hand or use a tank of compressed air or helium to inflate the balloon, which then has to be tied. This sequential filling is time consuming. In another example, an individual may fill water balloons with water by hand one at a time, and then tie the balloons, which can all be quite time-consuming. Moreover, the inflatable containers may be damaged or filled to different volumes.

US20080121309 discloses a balloon filling system for simultaneously filling a plurality of balloons. In US 20130118640 A1 a tie-less water balloon assembly is described, where the balloon includes an inner membrane for creating a one-way water flow channel. The channel includes a seal and closure member.

A method for inflating a balloon is found in FR 2911512 A1, where the method involves inserting all but the mouth portion of a balloon through the inside of an inflation lumen, folding the mouth of the balloon back over the outside of the inflation lumen, positioning an elastically deformable means over the folded back portion, then inflating and removing the balloon from the inflation lumen.

US patent 6,488,557 can be characterised as relating to a balloon inflation station having a needle able to be actuated to move through a resilient plug associated with the inflation station, in order to provide a pressurised gas to a balloon engaged with the resilient plug.

In US 20130226219 A1 an intragastric device and device for inflating and methods of fabricating, deploying, inflating, monitoring and retrieving the intragastric device are provided. The intragastric device comprises a balloon which is inflated by a catheter after the balloon is swallowed by a patient.

It is therefore an object of the present invention to provide an apparatus, system and method for filling containers with fluids that addresses at least some of the above mentioned disadvantages and/or that will at least provide the public with a useful choice.

### BRIEF DESCRIPTION OF THE INVENTION

Accordingly in a first aspect the present invention, there is provided an apparatus according to claim 1.

Preferably the apparatus is configured to fill the containers substantially simultaneously with a fluid.

Preferably the first end of the housing has an outermost perimeter that is smaller in length than an outermost perimeter of the second end.

Preferably the threaded opening at the first end of the housing has a threaded inner surface.

Preferably each container comprises an expandable balloon portion.

Preferably each container comprises a rigid portion and a flexible portion, the flexible portion disposed between the elastic fastener and the end of a respective one of the plurality of tubes.

Preferably each container comprises a volumetric measurement marking providing a visual reference for filling the container to a desired volume.

Preferably the elastic fastener is disposed outwardly from the container and clamps an inner surface of the container against an outer surface of the respective one of the plurality of tubes.

Preferably the fluid comprises one or more of water, air, and helium.

Preferably each elastic fastener comprises an o-ring configured to automatically seal the container in response to a force applied to the container in a direction away from the housing.

Preferably the plurality of tubes comprise a first set of tubes each having a first length, and a second set of tubes each having a second length longer than the first length.

Preferably the housing is attached to a valve coupled to a fluid source, wherein the valve is configured to control delivery of the fluid to fill the plurality of containers.

Preferably the valve includes a lever that can be turned to a first position to turn on the valve and allow fluid flow to the housing, wherein the lever can be turned to a second position to turn off the valve and stop fluid flow to the housing.

Preferably one end of the valve is connected to a hose attached to a water supply, and the other end is threaded to the housing.

In a second aspect the present invention, there is provided a method according to claim 10.

Preferably further comprising detaching the plurality of containers from the plurality of hollow tubes, wherein when each container is detached from the corresponding hollow tube, the elastic fastener seals the containers with the fluid inside.

Preferably the detaching comprises shaking the housing until the plurality of containers slip down the hollow tubes.

Preferably the detaching comprises pulling away the containers from the hollow tubes.

Preferably when the filled containers reach a threshold weight, the containers slip off the hollow tubes.

In yet a further aspect not part of the claimed invention may be said to be an apparatus comprising a tube removably joined to at least one elastic container by an open elastic valve, wherein the tube facilitates filling the elastic container with a fluid, the elastic valve being configured to automatically close upon removal of the tube from the elastic container to seal the fluid inside the elastic container.

Preferably the elastic valve comprises an elastic ring disposed around a neck of the elastic container.

In still a further aspect not part of the claimed invention may be said to be an apparatus comprising a plurality of flexible tubes, each flexible tube joined to a balloon, wherein the flexible tubes facilitate filling the balloons with a fluid, wherein the balloons are disposed in sufficient proximity to one another to push on each other during their filling, thereby causing the tubes to flex.

Preferably the flexible tubes are of different lengths.

In still a further aspect not part of the claimed invention may be said to be a balloon filling apparatus comprising a plurality of tubes and a plurality of balloons, wherein each balloon is connected to a respective one of the tubes with a connecting force not less than a weight equivalent to one of the balloons if substantially filled with water, the connecting force being overcomeable by applying an upward acceleration on the tube, wherein the apparatus is configured to fill the plurality of the balloons substantially simultaneously.

Preferably the connecting force is less than 1 Newton.

Preferably the connecting force is provided by an elastic valve configured to automatically seal the balloon if the connecting force is overcome.

Preferably each hole of the plurality of holes at the second end of the housing extends through an outer surface of the housing, the outer surface opposing the opening at the first end of the housing.

In still a further aspect not part of the claimed invention may be said to be an apparatus comprising or including
a conduit or manifold with at least one outlet and with an inlet for fluid to be ducted to egress from the or each outlet, and
a resiliently collared, resiliently banded and/or resiliently beaded fluid inflatable container constrained by the collar, band and/or bead such that its fluid entranceway is releasably about, but sealed to, the or its outlet;
the arrangement being that fluid can pass through such conduit or manifold in use to inflate the container(s) whilst the collar, band and/or bead of the or each container keeps the fluid entranceway about and sealed to the or its outlet;
and the arrangement further being such that the fluid content weight and/or inertia can assist or cause the release by axial separation of the fluid entranceway of the or each container from the or its outlet whereupon the or each collar, band and/or bead, no longer expanded by the or its tubular outlet, closes the or its fluid entranceway.

Preferably the outlet is tubular.

In yet a further aspect not part of the claimed invention may be said to be an apparatus comprising or including
a manifold or manifold assembly ('manifold') providing an ability to duct fluid ingressing via an inlet to egress out of each of a plurality of outlets,
a plurality of fluid inflatable containers each having its entranceway about a said outlet, and
a resilient collar, band and/or bead of and/or about each entranceway in a stretched condition thereby to hold the entranceway sufficiently tightly about its outlet to receive a fluid fill but not so tightly that the fluid fill weight and/or inertia cannot assist and/or cause the release and/or extraction of its outlet and thereafter the fluid tight sealing of the tubular entranceway by a contraction of its collar, band and/or bead.

The inlet is a threaded inlet.

Preferably the outlets are each tubular.

Preferably the entrance way is tubular.

In yet a further aspect not part of the claimed invention may be said to be a water balloon assembly comprising
a plurality of balloons each with a tubular entranceway, and
a collar, band or bead about or of each tubular entranceway, and
a manifold or manifold assembly ('manifold') having an inlet to receive water and duct such water out of each of a plurality of tubular outlets;
**wherein** each balloon is held sufficiently tightly by the collar, band or bead with the liquid entranceway about its tubular outlet of the manifold that water will pass out of its tubular outlet through its tubular entranceway to inflate the balloon;
**and wherein** each balloon can have its tubular entranceway sealed by a contraction of its collar, band or bead when no longer held to it's tubular outlet;
**and wherein** separation of a water inflated balloon from its manifold outlet can be caused or assisted by the weight or inertia of the water in the balloon.

In yet a further aspect not part of the claimed invention may be said to be an apparatus comprising or including
a plurality of resiliently chokered water inflatable balloons each held by its choker about its tubular entranceway, and as a sleeve, on an outlet tube of a filling manifold connectable at its inlet to a water source.

Preferably the choker hold on each manifold outlet enables post water inflation axial separation of the sleeved tubular entranceway from its tubular outlet and subsequent choker sealing of the tubular entranceway of the water inflated balloon.

Preferably the weight and/or inertia of the water content assists and/or causes the axial separation.

In yet a further aspect not part of the claimed invention may be said to be a filling procedure for multiple balloons, each to be sealed by a constrictor about its tubular entranceway, the procedure comprising the steps of
providing a manifold with an inlet to receive water and multiple tubular outlets, each outlet having a tubular entranceway, as a sleeve, held by the constrictor on the tubular entranceway, and
inflating each balloon with water and allowing drop off and/or causing drop off of each balloon to self seal reliant on its constrictor.

In even a further aspect not part of the claimed invention may be said to be a water filled balloon sealed by a constrictor about its tubular entranceway.

In even a further aspect not part of the claimed invention may be said to be a plurality of juxtaposed water filled balloons each in fluid communication via a manifold to a single water supply conduit.

In even a further aspect not part of the claimed invention may also be said to be a water filled balloon sealed by a constrictor about its tubular entranceway, such constrictor having effected the seal after a sliding off of the tubular entranceway from a filling spigot.

Preferably the filling spigot was a tubular outlet of a manifold thread engageable to a suitable tap.

In even a further aspect not part of the claimed invention may also be said to be a water filled balloon sealed by a constrictor about its beaded tubular entranceway, the beading assisting constrictor location about the entranceway.

In yet a further aspect not part of the claimed invention may be said to be an item of commerce comprising
(1) apparatus as herein before described and
(2) packaging that has the apparatus therein so as not to allow easy separation of each balloon or container from its associated tubular outlet from the manifold.

Preferably the apparatus allows a thread engagement of the inlet of the manifold to complementary faucet without separation of any balloon or container from the manifold.

In even a further aspect not part of the claimed invention may be said to be an apparatus suitable to provide a multiple of water filled and choker or constrictor sealed balloons upon water filling of the balloons using a manifold of the apparatus, the apparatus comprising a said manifold with a inlet to receive water and multiple tubular outlets out which water is to issue, a balloon docked about each tubular outlet to receive issuing water, and a said choker or constrictor of each balloon to serve the multiple functions:
(a) hold its balloon to receive issuing water,
(b) allow dedocking of its balloon,and
(c) choke or constrictor seal water content in its balloon as the water filled balloon is being dedocked and/or upon dedocking.

Preferably the hollow tubes as herein before described are rigidly flexible.

Preferably the tubes are able to bend, yet keep their non bent cross sectional shape.

Preferably the balloons, in their pre-filled condition, allow the distal ends of the tubes at where the balloons are engaged, to be in more condensed configuration compared to when the balloons filled.

Preferably the tubes extend substantially parallel each other and are adapted and configured, when being filled, to progressively splay outwards.

Preferably the tubes extend from the housing generally in the same direction and are adapted and configured, when being filled, to progressively splay outwards.

Preferably the tubes are adapted to not crease, fold or buckle when the balloons are being filled.

Preferably the outward splay is as a result of the expansion of the girth of the balloons as they are filled and the balloons being in contact with adjacent of said balloons.

Preferably the balloons do not contain any liquid prior to being filled with water.

Preferably the balloons contain air prior to being filled with water.

Preferably the air in the balloons is in fluid connection with ambient air.

Preferably there is no pressure differential between the air in the balloons and ambient air.

Preferably balloons, prior to being filled with water, are flaccid.

Preferably the balloons, once filled, are volumetrically, at least 3 times their flaccid size.

Preferably the balloons, once filled, are volumetrically, at least 5 times their flaccid size.

Preferably the balloons, once fully filled, are no more than 0.002 cubic metres.

Preferably the balloons, once fully filled, are no more than 0.0014 cubic metres.

Preferably the balloons, once fully filled, are no more than 0.0005 cubic metres.

Preferably the balloons, once fully filled, are no more than 0.0001 cubic metres.

Preferably the balloons comprise of a flexible body and a less flexible spout, the spout being engaged to a respective tube.

Preferably the flexible body or each balloon is located beyond the distal end of a respective tube.

Preferably a first group of tubes have their distal ends more proximate the housing/manifold than a second group of tubes, thereby locating a first group of balloons more proximate the housing/manifold than a second group of balloons.

Preferably the first group of tubes is located about the second group of tubes.

Preferably the tubes are arranged, at the housing/manifold in arrays of concentric circles.

Preferably the second group of tubes are of inner more concentric circles to the first group of tubes.

Preferably the ends of the tubes displace, upon filling of the balloons, to be more than 20mm apart each other.

Preferably the ends of the tubes displace, upon filling of the balloons, to be more than 25mm apart each other.

Preferably the ends of the tubes displace, upon filling of the balloons, to be more than 30mm apart each other.

Preferably the ends of the tubes displace, upon filling of the balloons, to be more than 35mm apart each other.

Preferably the ends of the tubes displace, upon filling of the balloons, to be more than 40mm apart each other.

Preferably the ends of the tubes displace, upon filling of the balloons, to be more than 45mm apart each other.

Preferably the ends of the tubes displace, upon filling of the balloons, to be more than 50mm apart each other.

Preferably the ends of the tubes displace, upon filling of the balloons, to be more than 55mm apart each other.

Preferably the ends of the tubes displace, upon filling of the balloons, to be more than 60mm apart each other.

Preferably the ends of the tubes displace, upon filling of the balloons, to be more than 65mm apart each other.

Preferably the ends of the tubes displace, upon filling of the balloons, to be no more than 80mm apart each other.

Preferably the ends of the tubes displace, upon filling of the balloons, to be no more than 70mm apart each other.

Preferably the ends of the tubes displace, upon filling of the balloons, to be no more than 65mm apart each other.

Preferably the ends of the tubes, when the balloons are flaccid, have a spacing of less than 50mm.

Preferably the ends of the tubes, when the balloons are flaccid, have a spacing of less than 40mm.

Preferably the ends of the tubes, when the balloons are flaccid, have a spacing of less than 30mm.

Preferably the ends of the tubes, when the balloons are flaccid, have a spacing of less than 20mm.

### BRIEF DESCRIPTION OF THE DRAWINGS

To provide a more complete understanding of the present disclosure and features and advantages thereof, reference is made to the following description, taken in conjunction with the accompanying figures, wherein like reference numerals represent like parts, in which:
FIGURE 1 is a simplified perspective view illustrating an example configuration of an embodiment of a system for filling containers with fluids;
FIGURE 2 is a simplified diagram illustrating a cross-sectional view of example details of an embodiment of the system;
FIGURE 3 is a simplified diagram illustrating other example details of an embodiment of the system;
FIGURE 4 is a simplified diagram illustrating yet other example details of an embodiment of the system;
FIGURE 5 is a simplified diagram illustrating yet other example details of an embodiment of the system;
FIGURE 6 is a simplified diagram illustrating yet other example details of an embodiment of the system;
FIGURE 7 is a simplified diagram illustrating yet other example details of an embodiment of the system; and
FIGURE 8 is a simplified flow diagram illustrating example operations that may be associated with an embodiment of the system.

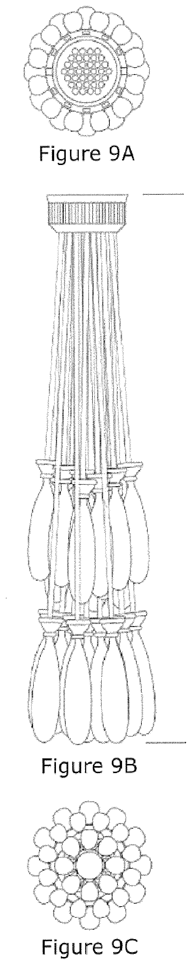

FIGURE 9a is an end view of figure 9b
FIGURE 9b is a side view of an example of the present invention
FIGURE 9c is an opposite end view of figure 9b

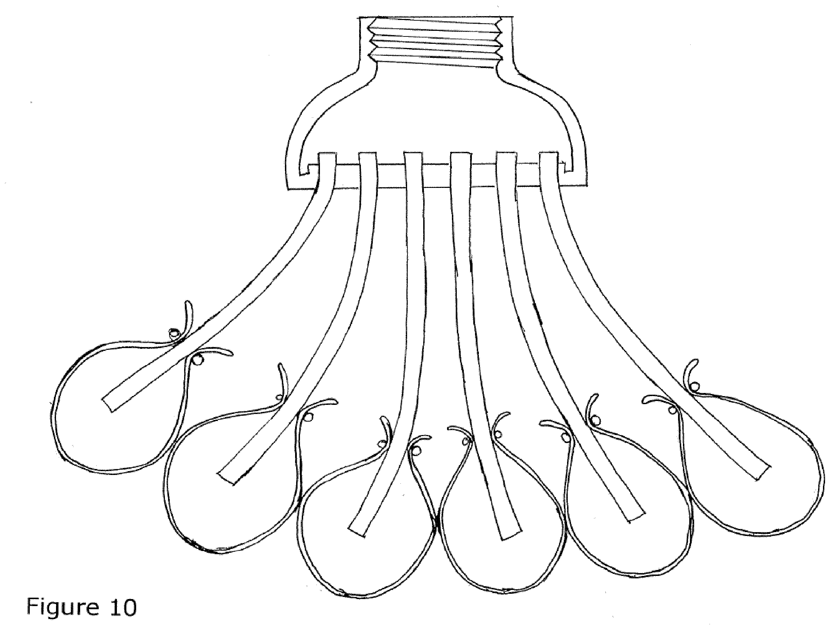

FIGURE 10 is a side view showing the tubes splayed apart more when the balloons have been filled.

### DETAILED DESCRIPTION OF THE INVENTION

In a broad sense the present invention includes or utilizes an apparatus that includes a housing (e.g., casing, manifold, covering, etc. that includes a cavity inside) with a first opening at a first region and a plurality of openings at a second region. , A plurality of hollow tubes extend from the plurality of openings to a plurality of containers (e.g., receptacles, vessels, ampules, test-tubes, balloons, etc.). The tubes may be integrally formed with the housing or separately assembled therewith. The containers are removably attached to the hollow tubes. A plurality of elastic fasteners are utilized Each elastic fastener clampsa container to a corresponding hollow tube, such that when the containers are filled with fluid and detached from the corresponding hollow tubes, each elastic fastener seals each container to help retain the fluid inside the container. The fasteners may be integrally formed with the container or separately associated therewith.

Some examples of the present invention will now be described with reference to the accompanying drawings. It is to be understood that the following disclosure describes several example embodiments for implementing different features, structures, or functions of the system. Example embodiments of components, arrangements, and configurations are described herein to simplify the present disclosure. However, these example embodiments are provided merely as examples and are not intended to limit the scope of the invention.

The present disclosure may repeat reference numerals and/or letters in the various exemplary embodiments and across the Figures provided herein. This repetitions is for the purpose of simplicity and clarity and does not in itself indicate a relationship between the various exemplary embodiments and/or configurations discussed in the various Figures.

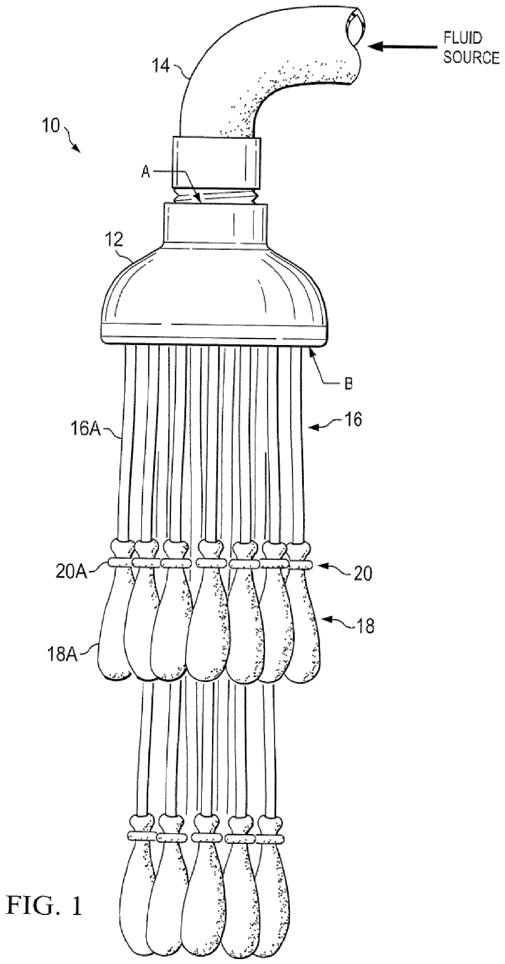

FIGURE 1 is a simplified diagram illustrating an example embodiment of a system 10 for filling containers with fluids. System 10 includes a housing 12 removably attachable to a hose 14 (e.g., tube, pipe, etc.) at a first region such as on a first end A and to a plurality of hollow tubes 16 at a second region such as the second end B.

As used herein, the term "housing" encompasses a hollow space or chamber enclosed by a rigid or semi-rigid casing (e.g., covering, skin, sleeve, sheath, etc.). A manifold arrangement is provide by the housing. End A includes a threaded opening configured to mate with corresponding threads on hose 14. In some embodiments, end A may be smaller in circumference or area than end B. Hose 14 may be connected to a fluid source, such as a water tank, gas tank, water supply line, etc. on end A. End B may include a plurality of openings such as holes (preferably configured in an array), configured to fit tubes 16. In some embodiments, tubes 16 may be integrally formed with the housing or are permanently attached (e.g., welded, brazed, stuck with adhesives, press-fitted, etc.) to housing 12. In other embodiments, tubes 16 may be removably attached (e.g., with threads, pressure, etc.) to housing 12.

The tubes each have an outlet opening distal the housing. A plurality of containers 18 may be held (e.g., attached, fastened, camped, clinched, secured, etc.) to plurality of hollow tubes 16 at the distal ends of the tube. This is preferably achieved using elastic valves 20. Each tube has one container associated with it.

As used herein, the term "container" refers to an object that can hold something, such as fluids. It is preferably an elastic container but does not need to be so. The term "valve" refers to an object that regulates, directs, or controls the flow of fluids, by opening, closing, or partially obstructing passageways of fluid flow. In an example embodiment, elastic valves 20 comprise elastic fasteners, such as O-rings or rubber bands. In another example embodiments, elastic valves 20 comprise corrugations, smocking, elastic fibers, etc. fabricated into the necks of containers 18. They are adapted and configured so that force is required to pull open the necks of containers 18, and removal of the force causes the necks to constrict and close. In yet another example embodiment, elastic valves comprise internal or external plugs affixed to the necks of containers 18, through which tubes 16 may be pushed through to clamp containers 18 thereto.

Note that each of container 18 hasan opening to facilitate clamping to tubes 16 and a cavity for containing fluid. For example, one end of an example tube 16A may be fitted through a hole in end B of housing 12, and the other end of tube 16A may be inserted into an example container 18A. An example elastic valve 20A (e.g., O-ring, comprising a mechanical gasket typically in a toroid shape; elastic ring, such as a rubber-band) of sufficient size to expand and clamp around tube 16A may be dispose around (e.g., placed over) a neck (e.g., portion just below opening) of container 18A, clamping and sealing container 18A to tube 16A. Thus, elastic valve 20A may be in an open configuration when container 18A is attached to tube 16A; in elastic valve 20A's open configuration, the neck of container 18A is open, allowing container 18A to fill with fluid. After container 18A is filled with fluid, it may be removed from tube 16A, whereupon elastic valve 20A is free to close and closes, thereby closing the neck of container 18A and sealing the fluid inside.

In one example embodiment, containers 18 may comprise inflatable balloons that may be filled with fluids such as water, air or helium. In another example embodiment, containers 18 may comprise flexible (e.g., stretchy, springy, etc.) elastic containers that may be filled with gaseous or liquid medications. As used herein, the term "elastic" is meant to refer to a property of a material that allows the material to resume its normal shape spontaneously after contraction, dilation, or distortion. In an example, an elastic material may be stretched to 200% of its original length, and the material may return to its original length when the stretching force is removed.

It will be appreciated that the valve may not make a complete seal of the container. An gas or liquid tight seal is preferred but some, minimal, leakage of fluid from the container may occur after removal from the tubes. Any leakage is preferably minimal for the applications to which the container is subsequently put. Eg if its for decorative balloons, slow leakage over a matter of days is likely to be of no consequence. Leakage may also be temporary in that subsequent additional sealing by other steps may take place. Eg decorative balloons may be clipped to a stick that has some sealing functionality to offer additional sealing for the balloon.

In yet another example embodiment, containers 18 may comprise flexible containers that may be filled with body fluids (e.g., urine, blood) for example, to collect multiple samples simultaneously for testing. Virtually any type and kind of fluid may be used within the broad scope of the embodiments. Note that in some embodiments, containers 18 need not be inflatable or flexible in their entireties. For example, a bottom portion of containers 18 may be inelastic (e.g., glass, plastic, metal, etc., of fixed shape and size), and a top portion may be flexible enough to be inserted around tubes 16 and clamped thereon.

When the fluid source is turned on, fluid may flow through housing 12, tubes 16 and fill containers 18. In some embodiments, when housing 12 is connected to a stream of liquid, containers 18 may be filled with the liquid. In some embodiments, the fluid may be supplied at high pressure. Virtually any mechanism that facilitates fluid flow through tubes 16 at sufficient pressure to fill containers 18 may be used within the broad scope of the embodiments. After containers 18 have reached a desired size or volume, they may be detached from tubes 16. In one example embodiment, filled containers 18 may be detached by pulling them away from tubes 16.

In another example embodiment, the connecting force holding filled containers 18 to tubes 16 may be overcome by an upward acceleration on tubes 16, for example, when they are shaken. Thus, filled containers 18 may be detached by shaking housing 12 (or tubes 16) sufficiently vigorously to cause containers 18 to fall off from tubes 16. In some embodiments, the connecting force holding filled container to its corresponding tube is not less than the weight of the filled container; in a specific embodiment, the connecting force holding each container to its corresponding tube is exactly equal to the weight of the filled container. The connecting force may be provided by a combination of constricting forces and friction forces from elastic valves 20.

In yet other embodiments, containers 18 may fall off under gravity; for example, when filled containers 18 reach a threshold weight, they slip off tubes 16 due to gravity. The threshold weight may be based upon the tightness of elastic valves 20, friction between tubes 16 and containers 18, and force from the weight of containers 18 (among other parameters). In various embodiments, containers 14 may slide off tubes 16 and elastic valves 20 may constrict the necks of containers 18, sealing them. In some embodiments, containers 18 may be marked with volumetric measurements, and fluid flow may be turned off when the fluid has filled containers 18 to a desired volume.

The end of the hollow tubes may include a flared outer lip or gradual taper increasing towards the end, that may present a hurdle for the elastic valve when sliding off the tube. An increased force may be applied to the container to overcome this hurdle prior to release from the tube. This may be desirable in order to prevent accidental or premature removal. However selection of an appropriate materials and their co-efficient of friction for the tubes and containers and an appropriately elastic valve, accidental or premature release may also be avoided.

In some embodiments, hollow tubes 16 may be made of a rigid material (e.g., steel, glass); in other embodiments, tubes 16 may be made of a flexible material (e.g., thin plastic). In some embodiments, tubes 16 may be thick, short and rigid; in other embodiments, tubes 16 may be slender, long and flexible. Thus, hollow tubes 16 may be flexible, semi-rigid, or rigid, based on its material of construction, design, or a combination thereof. Note that tubes 16 may be of different lengths, for example, to prevent crowding and to accommodate a larger number of containers 18 than would be possible if tubes 16 were of the same length. Thus, at least some of hollow tubes 16 may be of different lengths than the others.

Also, tubes 16 may be flexible to enable containers 18 to expand. Thus, as containers 18 fill with fluid and expand, they may push against each other, flexing tubes 16. The outermost tubes 16 may be flexed more than the innermost tubes 16 (outer and inner being in reference to a center-point of housing 12, with the inner tubes 16 being closer to the center-point, and the outer tubes 16 being farther from the center-point).

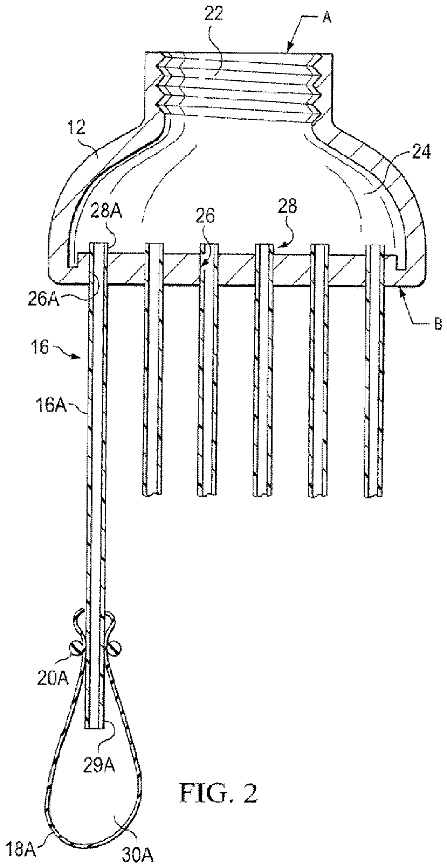

Turning to FIGURE 2, FIGURE 2 is a simplified cross-sectional view of a portion of an embodiment of system 10. Housing 12 comprises a threaded opening 22 at end A, an internal cavity 24, and an array of holes 26 at end B. Internal cavity 24 facilitates distributing the fluid entering at threaded opening 22 to array of holes 26 at end B. In some embodiments, threaded opening 22 may be configured for attaching to a fluid supply hose 14 (e.g., garden hose, plastic tube, etc.). In other embodiments, threaded opening 22 may be attached to corresponding threads in a valve. Array of holes 26 may be configured for connecting first ends 28 of tubes 16 by any suitable means. In some embodiments, first ends 28 of tubes 16 may be connected to corresponding holes 26 by compressing or gluing. In some embodiments, a number of holes 26 in housing 12 and a number of tubes 16 can correspond to a number of containers 18 that are desired to be filled and sealed substantially simultaneously.

To clarify further, only one example tube 16A is shown in the figure. A first end 28A of tube 16A is fitted through a corresponding hole 26A in housing 12. A second end 29A of tube 16A is inserted into container 18A. Elastic valve 20A may be placed around the neck of container 18A clamping the neck to tube 16A. An internal volume 30A of container 18A may be filled with fluid appropriately.

To fill and seal containers 18, housing 12 may be attached to a fluid supply tube (e.g., garden hose) and the fluid supply may be turned on. The fluid enters housing 12, is distributed to holes 26, travels down tubes 16, and fills containers 18. Containers 18 may be filled and may expand substantially simultaneously. When containers 18 have reached a desired size and/or they are filled with the desired volume of fluid, they may be removed from tubes 16. They can be removed by falling off, by shaking them off, by pulling them off by hand, etc. As each container 18A is removed from corresponding tube 16A, respective elastic valve 20A may constrict and close the neck of container 18A, sealing it with the fluid inside.

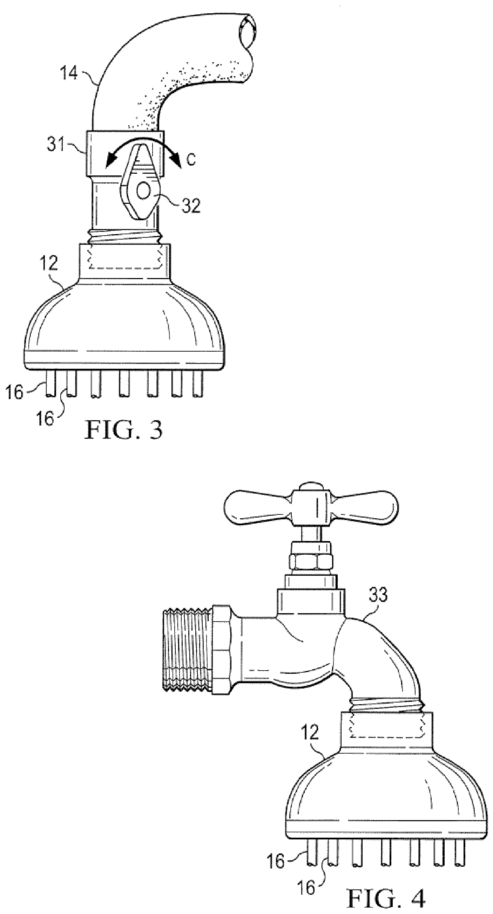

Turning to FIGURE 3, FIGURE 3 is a simplified diagram illustrating example details of a valve 31 that may be attached between hose 14 and housing 12 according to an embodiment of system 10. One end of valve 31 may be attached to hose 14 and the other end may be attached to threaded opening 22 of housing 12 (e.g., using threads). A lever 32 may be turned from one side (of valve 31) to another (e.g., as indicated by arrow C) to turn on and turn off fluid flow to housing 12. For example, to turn on the fluid flow, lever 32 may be turned to a first position; lever 32 may be turned to a second position (e.g., different from the first position) to turn off fluid flow.

Turning to FIGURE 4, FIGURE 4 is a simplified diagram illustrating example details of an embodiment of system 10. Housing 12 may be attached to a spigot 33 (e.g., nozzle, faucet, outlet, etc.) that connects to the fluid source. Spigot 33 may be turned on or turned off to start or stop fluid flow to housing 12.

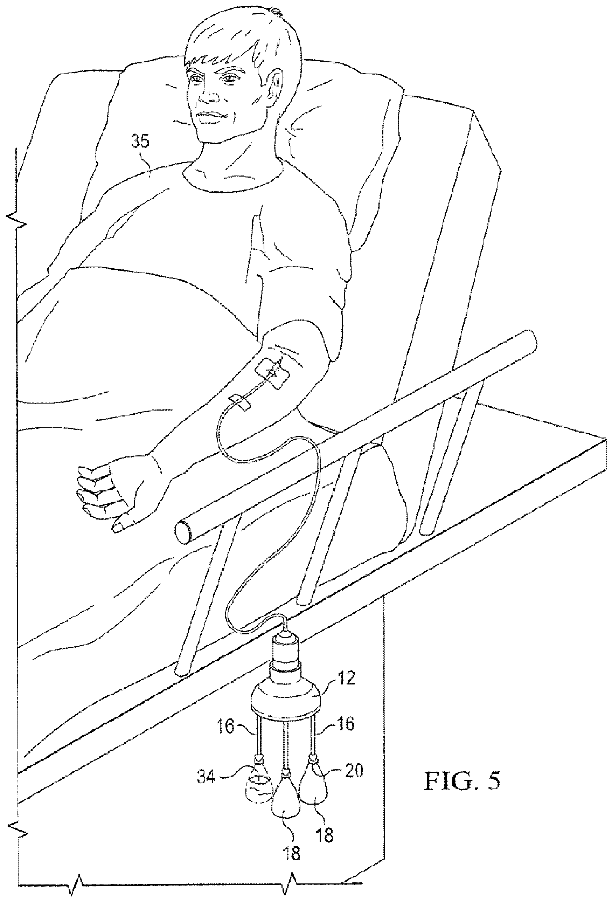

Turning to FIGURE 5, FIGURE 5 is a simplified diagram illustrating example details of an application of an embodiment of system 10. Embodiments of system 10 may be used in a variety of applications, such as for collecting numerous blood samples substantially simultaneously. Blood 34 may be drawn from a human (or animal) and blood 34 may collect substantially simultaneously in plurality of containers 18. The substantial simultaneous collection of blood in such manner can ease patient pain, speed up sampling time, and enable taking multiple samples substantially simultaneously without cross-contamination from one container to another or messy transfers between containers.

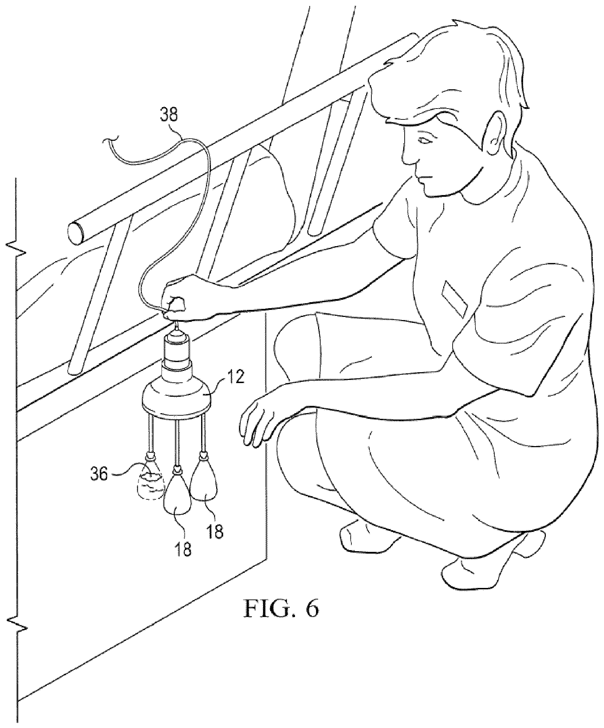

Turning to FIGURE 6, FIGURE 6 is a simplified diagram illustrating example details of an application of an embodiment of system 10. Embodiments of system 10 may be used in a variety of applications, such as for collecting numerous urine samples substantially simultaneously. Urine 36 may be drawn from a human (or animal) through a suitable catheter 38, and may collect substantially simultaneously in plurality of containers 18.

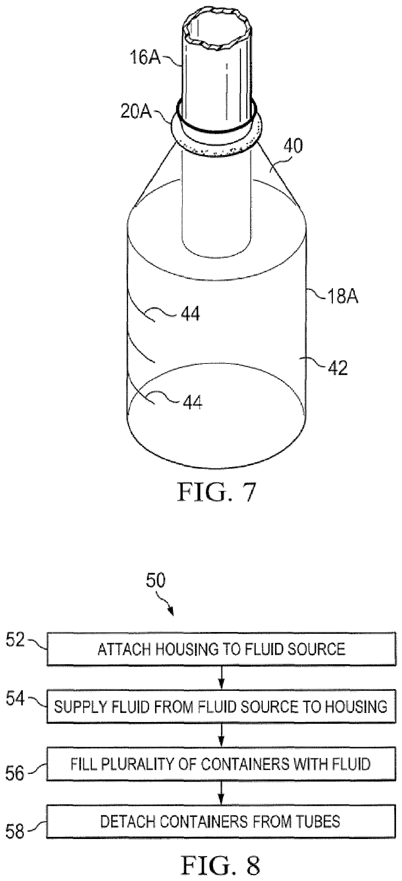

Turning to FIGURE 7, FIGURE 7 is a simplified diagram illustrating example details of an embodiment of system 10. Example container 18A may comprise a flexible portion 40 and an inflexible portion 42. Flexible portion 40 may be clamped on to example tube 16A using example elastic valve 20A. In some embodiments, container 18A may comprise volumetric measurement markings 44. When fluid fills container 18A to a desired volume, for example, as indicated by volumetric measurement marking 44, container 18A may be detached from tube 16A, whereupon elastic valve 20A may close container 18A, sealing the fluid inside.

Turning to FIGURE 8, FIGURE 8 is a simplified flow diagram 50 illustrating example operations that may be associated with an embodiment of system 10. At 52, housing 12 may be attached to a fluid source (e.g., through hose 14, spigot 33, etc.) At 54, fluid may be supplied from the fluid source to housing 12. At 56, plurality of containers 18 may be filled with the fluid. At 58, containers 18 may be detached from corresponding tubes 16.

Note that in this Specification, references to various features (e.g., elements, structures, modules, components, steps, operations, characteristics, etc.) included in "one embodiment", "example embodiment", "an embodiment", "another embodiment", "some embodiments", "various embodiments", "other embodiments", "alternative embodiment", and the like are intended to mean that any such features are included in one or more embodiments of the present disclosure, but may or may not necessarily be combined in the same embodiments.

The elements described herein may be made of any suitable materials, including metal (e.g., stainless steel, copper, brass, bronze, aluminum, etc.), plastic, glass, elastomers, or any suitable combination thereof. Each element may also be made of a combination of different materials (e.g., housing and tubes may be made of plastic and containers may be made of elastic rubber; housing and tubes may be made of stainless steel and containers may be made of a combination of glass and flexible plastic; etc.). Any suitable material or combination of materials may be used for the components described herein without departing from the broad scope of the present disclosure.

In addition, the shapes shown and illustrated in the various FIGURES are for example purposes only. Various other shapes may be used herein without changing the scope of the present disclosure. For example, housing 12 may be conical, cylindrical, pyramidal, etc., without departing from the broad scope of the embodiments. Likewise, tubes 16 may be rigid, or flexible 18 without departing from the scope of the broad embodiments.

While the disclosure references several particular embodiments, those skilled in the art will be able to make various modifications to the described embodiments without departing from the scope of the invention which is defined by the appended claims.

## Claims

1. An apparatus comprising:
a housing (12) comprising a threaded opening at a first end (A) and a plurality of holes (26) at a second end (B); and
a plurality of hollow tubes (16), wherein each hollow tube (16) is attached to a respective hole of said plurality of holes (26),
wherein all of the hollow tubes of the apparatus extend from said housing to respective tube ends at where the tube ends present a plurality of adjacent inflatable containers (18),
wherein each tube end is removably joined to a respective one of said inflatable containers in a sealed manner by an elastic ring (20) disposed around a neck of the inflatable container,
and wherein the tubes facilitate the simultaneous filling of the inflatable containers with a liquid,
said elastic rings being configured to each slide with the corresponding inflatable container when each inflatable container is detached from the corresponding hollow tube and, upon removal of the inflatable container from the corresponding tube, to constrict the neck of the inflatable container, sealing it with the liquid inside.

2. The apparatus of claim 1 wherein the outermost perimeter of the first end (A) of the housing (12) is smaller in length the outermost perimeter of the second end (B) of the housing (12).

3. The apparatus of claim 2 wherein the first end (A) of the housing (12) has a threaded inner surface (22) at the threaded opening.

4. The apparatus of any one of claims 1 to 3, wherein each inflatable container (18) comprises an expandable balloon portion.

5. The apparatus of any one of claims 1 to 4, wherein the elastic ring (20) is disposed outwardly from the inflatable container (18) and clamps an inner surface of the inflatable container against an outer surface of the respective one of the plurality of hollow tubes (16).

6. The apparatus of any one of claims 1 to 5, wherein the elastic rings (20) are configured to permit the inflatable containers (18) to be removed from the tubes (16) by falling off, or by manually shaking them off, when the containers (18) have reached the desired size.

7. The apparatus of claim 6, wherein the elastic rings (20) are configured to permit removal and sealing of the containers (18) substantially simultaneously.

8. The apparatus of claim 1, wherein each hole of the plurality of holes (26) at the second end (B) of the housing extends through an outer surface of the housing, the outer surface at the second end of the housing being located at an opposed end of the housing from the threaded opening at the first end (A) of the housing (12).

9. A method of filling a plurality of inflatable containers simultaneously with a liquid, comprising:
attaching a housing (12) to a liquid source, wherein the housing comprises a threaded liquid inlet and a plurality of holes (26) separated from the liquid inlet, and further comprises a plurality of hollow tubes (16) each hollow tube (16) being attached to a respective hole of the plurality of holes (26), all of the hollow tubes (16) extending from said housing (12), in a juxtaposed manner, to respective distal tube ends, each tube end providing a liquid outlet opening that is in liquid connection with the liquid inlet, said tube ends presenting a plurality of juxtaposed inflatable containers (18), each container being removably attached to a respective tube end of the plurality of hollow tubes and about the liquid outlet of its respective hollow tube end, wherein an elastic ring (20) clamps a neck of each inflatable container in a sealed manner to a corresponding one of the hollow tubes;
supplying a liquid from the liquid source to the housing via the liquid inlet,
substantially simultaneously filling the plurality of inflatable containers with the liquid,
and detaching the plurality of inflatable containers from the plurality of hollow tubes, wherein when each inflatable container is detached from the corresponding hollow tube, the elastic rings slide with their respective inflatable container and, upon removal of the inflatable containers from their respective tube, seal their respective inflatable container with the liquid inside by constricting the neck of their respective inflatable container.

10. The method of claim 9, wherein the detaching comprises shaking the housing (12) until the plurality of inflatable containers (18) slip down the hollow tubes (16).

## Patentansprüche

1. Vorrichtung, umfassend:
ein Gehäuse (12), das eine mit Gewinde versehene Öffnung an einem ersten Ende (A) und eine Vielzahl von Löchern (26) an einem zweiten Ende (B) umfasst; und
eine Vielzahl von Hohlrohren (16), wobei jedes Hohlrohr (16) an einem jeweiligen Loch der Vielzahl von Löchern (26) angebracht ist,
wobei sich alle der Hohlrohre der Vorrichtung aus dem Gehäuse zu jeweiligen Rohrenden erstrecken, dort wo die Rohrenden eine Vielzahl von angrenzenden aufblasbaren Behältern (18) präsentieren,
wobei jedes Rohrende entfernbar mit einem jeweiligen der aufblasbaren Behälter auf eine dichtbare Weise durch einen elastischen Ring (20) verbunden ist, der um einen Hals des aufblasbaren Behälters angeordnet ist,
und wobei die Rohre das gleichzeitige Befüllen der aufblasbaren Behälter mit einer Flüssigkeit erleichtern,
wobei die elastischen Ringe ausgelegt sind, jeweils mit dem entsprechenden aufblasbaren Behälter zu gleiten, wenn jeder aufblasbare Behälter vom entsprechenden Hohlrohr abgelöst wird und, nach dem Entfernen des aufblasbaren Behälters vom entsprechenden Rohr, den Hals des aufblasbaren Behälters zu verengen, um ihn mit der Flüssigkeit im Innern abzudichten.

2. Vorrichtung nach Anspruch 1, wobei der äußerste Umfang des ersten Endes (A) des Gehäuses (12) an Länge kleiner als der äußerste Umfang des zweiten Endes (B) des Gehäuses (12) ist.

3. Vorrichtung nach Anspruch 2, wobei das erste Ende (A) des Gehäuses (12) eine mit Gewinde versehene innere Oberfläche (22) an der mit Gewinde versehenen Öffnung aufweist.

4. Vorrichtung nach einem der Ansprüche 1 bis 3, wobei jeder aufblasbare Behälter (18) einen ausdehnbaren Ballonabschnitt umfasst.

5. Vorrichtung nach einem der Ansprüche 1 bis 4, wobei der elastische Ring (20) ab dem aufblasbaren Behälter (18) nach außen gerichtet angeordnet ist und eine innere Oberfläche des aufblasbaren Behälters gegen eine äußere Oberfläche des jeweiligen Rohrs der Vielzahl von Hohlrohren (16) klemmt.

6. Vorrichtung nach einem der Ansprüche 1 bis 5, wobei die elastischen Ringe (20) ausgelegt sind, zu erlauben, dass die aufblasbaren Behälter (18) durch Abfallen oder durch manuelles Abschütteln von den Rohren (16) entfernt werden, wenn die Behälter (18) die gewünschte Größe erreicht haben.

7. Vorrichtung nach Anspruch 6, wobei die elastischen Ringe (20) ausgelegt sind, das Entfernen und Abdichten der Behälter (18) im Wesentlichen gleichzeitig zu erlauben.

8. Vorrichtung nach Anspruch 1, wobei sich jedes Loch der Vielzahl von Löchern (26) am zweiten Ende (B) des Gehäuses durch eine äußere Oberfläche des Gehäuses erstreckt, wobei sich die äußere Oberfläche am zweiten Ende des Gehäuses am entgegengesetzten Ende des Gehäuses von der mit Gewinde versehenen Öffnung am ersten Ende (A) des Gehäuses (12) befindet.

9. Verfahren zum gleichzeitigen Befüllen einer Vielzahl von aufblasbaren Behältern mit einer Flüssigkeit, umfassend:
Anbringen eines Gehäuses (12) an eine Flüssigkeitsquelle, wobei das Gehäuse einen Flüssigkeitseinlass und eine Vielzahl von Löchern (26) aufweist, die vom Flüssigkeitseinlass getrennt sind, und ferner eine Vielzahl von Hohlrohren (16) umfasst, wobei jedes Hohlrohr (16) an einem jeweiligen Loch der Vielzahl von Löchern (26) angebracht ist, wobei sich alle der Hohlrohre (16) aus dem Gehäuse (12), auf eine nebeneinandergestellte Weise, zu jeweiligen fernen Rohrenden erstrecken, wobei jedes Rohrende eine Austrittsöffnung für Flüssigkeit bereitstellt, die in Flüssigkeitsverbindung mit dem Flüssigkeitseinlass steht, wobei die Rohrenden eine Vielzahl von nebeneinandergestellten aufblasbaren Behältern (18) präsentieren, wobei jeder Behälter entfernbar an einem jeweiligen Rohrende der Vielzahl von Hohlrohren und um den Flüssigkeitsauslass seines jeweiligen Hohlrohrendes angebracht ist, wobei ein elastischer Ring (20) einen Hals jedes aufblasbaren Behälters auf eine abgedichtete Weise an ein entsprechendes der Hohlrohre klemmt;
Zuführen einer Flüssigkeit von der Flüssigkeitsquelle über den Flüssigkeitseinlass zum Gehäuse,
im Wesentlichen gleichzeitiges Befüllen der Vielzahl von aufblasbaren Behältern mit der Flüssigkeit,
und Ablösen der Vielzahl von aufblasbaren Behältern von der Vielzahl von Hohlrohren, wobei, wenn jeder aufblasbare Behälter vom entsprechenden Hohlrohr abgelöst wird, die elastischen Ringe mit ihrem jeweiligen aufblasbaren Behälter gleiten und, nach dem Entfernen der aufblasbaren Behälter von ihrem jeweiligen Rohr, ihren jeweiligen Behälter mit der darin befindlichen Flüssigkeit abdichten, indem der Hals ihres jeweiligen aufblasbaren Behälters verengt wird.

10. Verfahren nach Anspruch 9, wobei das Ablösen das Schütteln des Gehäuses (12) umfasst, bis die Vielzahl von aufblasbaren Behältern (18) an den Hohlrohren (16) herabgleitet.

## Revendications

1. Appareil comprenant :
un carter (12) comprenant une ouverture filetée à une première extrémité (A) et une pluralité de trous (26) à une seconde extrémité (B) ; et
une pluralité de tubes creux (16), dans lequel chaque tube creux (16) est fixé à un trou respectif de ladite pluralité de trous (26),
dans lequel tous les tubes creux de l'appareil s'étendent à partir dudit carter vers les extrémités respectives des tubes où les extrémités des tubes présentent une pluralité de récipients gonflables (18) adjacents,
dans lequel chaque extrémité de tube est raccordée de manière amovible à un récipient respectif desdits récipients gonflables de façon étanche au moyen d'une bague élastique (20) disposée autour du cou du récipient gonflable,
et dans lequel les tubes facilitent le remplissage simultané des récipients gonflables avec un liquide,
lesdites bagues élastiques étant configurées pour coulisser chacune avec le récipient gonflable correspondant lorsque chaque récipient gonflable est séparé du tube creux correspondant et, après le retrait du récipient gonflable à partir du tube correspondant, pour serrer le cou du récipient gonflable, pour le rendre étanche alors que le liquide se trouve à l'intérieur.

2. Appareil selon la revendication 1, dans lequel le périmètre extérieur de la première extrémité (A) du carter (12) est d'une longueur inférieure au périmètre extérieur de la seconde extrémité (B) du carter (12).

3. Appareil selon la revendication 2, dans lequel la première extrémité (A) du carter (12) comporte une surface interne filetée (22) au niveau de l'ouverture filetée.

4. Appareil selon l'une quelconque des revendications 1 à 3, dans lequel chaque récipient gonflable (18) comprend une partie de ballon extensible.

5. Appareil selon l'une quelconque des revendications 1 à 4, dans lequel la bague élastique (20) est disposée vers l'extérieur à partir du récipient gonflable (18) et serre une surface interne du récipient gonflable contre une surface externe du tube respectif de la pluralité de tubes creux (16).

6. Appareil selon l'une quelconque des revendications 1 à 5, dans lequel les bagues élastiques (20) sont configurées pour permettre aux récipients gonflables (18) de se détacher des tubes (16) en tombant ou en les secouant manuellement, dès que les récipients (18) ont atteint la taille souhaitée.

7. Appareil selon la revendication 6, dans lequel les bagues élastiques (20) sont configurées pour permettre le retrait et la fermeture hermétique des récipients (18) essentiellement en même temps.

8. Appareil selon la revendication 1, dans lequel chaque trou de la pluralité de trous (26) au niveau de la seconde extrémité (B) du carter s'étend à travers une surface externe du carter, la surface externe au niveau de la seconde extrémité du carter se trouvant à une extrémité du carter qui est opposée à l'ouverture filetée au niveau de la première extrémité (A) du carter (12).

9. Procédé de remplissage simultané d'une pluralité de récipients gonflables avec un liquide, comprenant les étapes consistant à :
attacher un carter (12) à une source de liquide, dans lequel le carter comprend une orifice fileté d'entrée de liquide et une pluralité de trous (26) séparés de l'orifice d'entrée de liquide, et comprend en outre une pluralité de tubes creux (16), chaque tube creux (16) étant fixé à un trou respectif de la pluralité de trous (26), tous les tubes creux (16) s'étendant à partir du carter (12), de manière juxtaposée, jusqu'aux extrémités distales respectives des tubes, chaque extrémité de tube servant d'ouverture de sortie de liquide qui est en contact liquide avec l'orifice d'entrée de liquide, lesdites extrémités des tubes présentant une pluralité de récipients gonflables (18) juxtaposés, chaque récipient étant raccordé de manière amovible à une extrémité de tube respectif de la pluralité de tubes creux et autour de la sortie de liquide de l'extrémité de son tube creux respectif, dans lequel une bague élastique (20) serre le cou de chaque récipient gonflable de manière étanche sur un tube correspondant des tubes creux ;
fournir, à partir de la source de liquide, un liquide au carter par l'intermédiaire de l'orifice d'entrée de liquide,
remplir de liquide essentiellement en même temps la pluralité de récipients gonflables, et
séparer la pluralité de récipients gonflables de la pluralité de tubes creux, dans lequel quand chaque récipient gonflable est séparé du tube creux correspondant, les bagues élastiques coulissent avec leur récipient gonflable respectif et, lors du retrait des récipients gonflables de leur tube respectif, rendent étanches leur récipient gonflable respectif, alors que le liquide se trouve à l'intérieur, en resserrant le cou de leur récipient gonflable respectif.

10. Procédé selon la revendication 9, dans lequel la séparation consiste à secouer le carter (12) jusqu'à ce que la pluralité de récipients gonflables (18) glissent et tombent des tubes creux (16).
